# EUROPEAN PATENT SPECIFICATION

(11) **EP 4 286 860 B1**
(45) Date of publication and mention of the grant of the patent: **13.08.2025**
(21) Application number: 22176146.3
(22) Date of filing: 30.05.2022
(51) Int. Cl.: G01P 13/00, G01P 15/00, B60Q 5/00, B60Q 9/00, G10K 15/02, A61M 21/00, G10K 11/178

(54) **A SYSTEM, A METHOD AND A COMPUTER PROGRAM PRODUCT FOR PROVIDING AN INDICATION OF AN ACCELERATION OF A VEHICLE FOR ROAD USE AND SUCH A VEHICLE FOR ROAD USE**
SYSTEM, VERFAHREN UND COMPUTERPROGRAMMPRODUKT ZUR ANZEIGE DER BESCHLEUNIGUNG EINES STRASSENFAHRZEUGS UND EIN SOLCHES STRASSENFAHRZEUG
SYSTÈME, PROCÉDÉ ET PRODUIT DE PROGRAMME INFORMATIQUE POUR FOURNIR UNE INDICATION DE L'ACCÉLÉRATION D'UN VÉHICULE ROUTIER ET UN TEL VÉHICULE ROUTIER

(43) Date of publication of application: 06.12.2023
(73) Proprietor: Bayerische Motoren Werke Aktiengesellschaft, 80809 München (DE)
(72) Inventor: Zaki, Tarek, 81829 München (DE)

(56) References cited:
- WO-A1-2012/053043
- JP-A- 2010 228 473
- US-A1- 2016 016 513
- US-A1- 2020 101 896

## Description

The present invention relates to a vehicle for road use and to a system and method for use in connection with such a vehicle. The invention further relates to a computer program for carrying out the method.

The present invention can, in principle, be used in connection with any type of vehicle for road use, in particular a motorized vehicle, such as a (passenger) car, lorry / truck, bus or coach. Given that the invention finds particular application in (passenger) cars, the invention will be described primarily in connection with cars, without being limited to this. In particular, one skilled in the art will not have any difficulties, guided by the present disclosure, to implement the invention in the context of vehicles other than cars.

A large number of people experience motion sickness (also referred to as kinetosis, among other names) when travelling in a vehicle. Typically, this problem is exacerbated if the person travelling in the vehicle and susceptible to motion sickness (in the following simply "person" or "user") looks at an object within the vehicle, in particular an object which is substantially still with respect to the vehicle, while the vehicle may accelerate and decelerate, both in a longitudinal direction (i.e. the main direction of travel) or in a transverse direction, for example when the vehicle passes through a curve or makes a turn (lateral acceleration) or passes over a speed bump or other uneven road surface etc. (vertical acceleration) or combinations thereof. As used herein, the term "acceleration", "accelerate" and similar is preferably intended to be understood to encompass not only acceleration, but also deceleration (effectively a negative acceleration), in any direction, unless the context specifies otherwise.

DE 10 2018 214 975 A1 discloses a system for visually indicating an anticipated acceleration of an autonomously driving vehicle to a person travelling in the vehicle. Information regarding the anticipated acceleration is derived from a navigation system of the autonomously driving vehicle. This document also briefly mentions the possibility of indicating the anticipated acceleration by means of an acoustic signal, however without disclosing any details regarding this idea.

US 2016/0016513 A1 discloses a feedback system that alters the distribution of a sound in a vehicle from a default configuration indicating to the driver that his action is dangerous or inefficient. The feedback system monitors one or more forces or accelerations such as speeding up, slowing down, changing the vehicle's direction, etc.

US 2020/0101896 A1 discloses a vehicle sound synthesis system with a loudspeaker and a controller. The loudspeaker projects sound indicative of synthesized engine noise (SEN) within a cabin of a vehicle in response to receiving a SEN signal.

WO 2012/053043 A1 discloses a pseudo sound signal generating unit which generates pseudo engine sound signals on the basis of the traveling information of a vehicle obtained by means of an acquisition unit.

It is an object of at least some embodiments of the present invention to provide an alternative technique of providing an indication of an acceleration of a vehicle, in particular an improved technique of providing an indication of an acceleration of a vehicle.

The invention is set out in the claims.

In a first aspect, the present disclosure provides a system for providing an indication of an acceleration of a vehicle for road use, the system comprising:
a detector configured to detect an actual acceleration of the vehicle and to provide a detection output based thereon;
a processing unit configured to receive the detection output, the processing unit further being configured to generate a control signal based thereon; and
an acoustic output device configured to receive the control signal and to generate and output an audible sound as a function of the control signal, the audible sound being indicative of the actual acceleration of the vehicle.

In the sense of the invention, the term "actual acceleration" refers not simply to a theoretical value of acceleration that the vehicle may achieve (sometimes expressed in terms of time taken to accelerate from 0 to 100 km per hour), but a current, actual acceleration of the vehicle in a physical sense. It is however to be appreciated that the term "actual acceleration" as detected by the detector does not need to be a value which (directly) provides the correct measurement of the actual acceleration as expressed in (correct) physical units, such as a value expressed in m/s². Instead, the term "actual acceleration" can, in principle, mean any information which (at least approximately) characterises the acceleration of the vehicle, in particular (at least approximately) uniquely characterises the acceleration of the vehicle, in particular a value or set of values from which the physically correct measurement of the actual acceleration can (at least approximately) be obtained, in particular without having to resort to other measurements. The actual acceleration as detected by the detector may in particular comprise a numerical value or values, in particular a set of two, three or more values (e.g. one value for each Cartesian direction), in particular a vector or information representing a vector. In particular, the actual acceleration as detected by the detector would be such that a computing device can process it.

Suitable detectors for detecting the actual acceleration of the vehicle are known in the art and include, for example, accelerometers and gyroscope sensors or combinations of these.

In the sense of the invention, a "processing unit" is preferably intended to be understood to mean any electrical component or device which can receive a detection output from the detector and generate a control signal based thereon which can be used by the acoustic output device. The processing unit can either (substantially) transparently pass the detection output to the acoustic output device if the latter can be controlled directly by the detection output. The processing unit may however process the detection output and generate a control signal which is (substantially) different from the detection output. The processing unit may in particular comprise a microprocessor. The processing unit of the first aspect of the present disclosure may, for example, comprise an onboard computer of the vehicle, or a component thereof.

Examples of acoustic output devices will be described below. Whilst the audible sound needs to be appreciable by a person, it does not necessarily need to be particularly loud. In particular, it may be output in addition to other sounds that can be heard in the vehicle, and the (average and/or peak) volume of the audible sound may be lower than the (average and/or peak) volume of such other sounds. The audible sound may be output by an acoustic output device which also outputs such other sounds, or it may be output by a different acoustic output device. Also, the audible sound may be a sound which, in the absence of any actual acceleration of the vehicle, would not be output by the acoustic output device, or it may be a modification of a sound which, even in the absence of any actual acceleration of the vehicle, would be output by the acoustic output device.

The system can be built into the vehicle or may form part of the vehicle. However, the system can also be provided on its own and, for example, be supplied to vehicle manufacturers so that the system may be built into vehicles.

Some embodiments of the first aspect of the present disclosure will now be described.

In some embodiments, the system is configured to modulate the audible sound with, or as a function of, the control signal.

In this way, the person can easily appreciate a (direct) correlation between the acceleration of the vehicle and the audible sound, which may help to alleviate the effects of motion sickness. For example, the modulation may be such that a physical property of the audible sound is varied linearly with a measure of the actual acceleration of the vehicle. However, the correlation between the measure of the actual acceleration and the physical property of the audible sound does not need to be linear. Instead, the physical property of the audible sound may vary as a function of the actual acceleration in such a way that it increases at a greater than linear rate, or alternatively at a smaller than linear rate. In any event, it is preferred that the correlation between the physical property of the audible sound and the actual acceleration is such that it can be represented by a monotone function, in particular a strict monotone function.

In some embodiments, the system is configured to modulate the audible sound with, or as a function of, the control signal in terms of frequency and/or volume and/or another property of the audible sound.

A modulation in terms of frequency and/or volume of the audible sound based on the actual acceleration can provide the person with an effective indication of the actual acceleration of the vehicle, in particular one that is easily recognised or "felt" as an indication of the actual acceleration of the vehicle. In addition, or as an alternative, other properties of the audible sound can also be modulated, such as a waveform of the audible sound.

In some embodiments, the audible sound is an audible sound arranged to be generated by the vehicle
- to indicate that the vehicle is operational and/or
- to indicate that the vehicle is moving and/or
- to resemble the sound of an internal combustion engine.

This may be particularly useful in connection with electric vehicles since they tend to be quieter than vehicles with an internal combustion engine, in particular when they are stationary. The audible sound may therefore be a (subtle) sound that is (artificially) generated or added to give the person an indication of the condition of the vehicle (operational/moving etc.). As indicated above, such a sound may be generated and output regardless of whether the vehicle undergoes any acceleration or not. The latter case may occur for example after the person has put the vehicle into an "activated state", e.g. by inserting a key, key card or similar into an appropriate receptacle of the vehicle or has pressed a start button or similar and while the vehicle remains stationary. Likewise, a condition without acceleration can be a case where the vehicle is moving in a straight line at a constant speed.

An audible sound of the type described above may however also be generated and output in connection with a vehicle which is equipped with an internal combustion engine, for example in a vehicle with noise cancellation function, where the noise generated by the engine might not (easily) be heard within the vehicle.

In the embodiments described above, the sound intended to provide the person with an indication of the condition of the vehicle (operational/moving etc.) can then be modified/modulated depending on the actual acceleration, for example by an increased volume of such a sound, or by superimposing another sound.

In some embodiments, the audible sound is composed of a sound of one or more musical instruments or processed versions thereof, wherein the processing unit is preferably configured to modify the sound to be output, by the acoustic output device, by:
- adding a sound of a further musical instrument or a processed version thereof upon detection of an acceleration of the vehicle, in particular an acceleration surpassing a predetermined or configurable threshold, in particular an acceleration of the vehicle in a first direction relative to the vehicle, and/or
- removing the sound of a musical instrument or a processed version thereof upon detection of a deceleration of the vehicle, in particular a deceleration surpassing a predetermined or configurable threshold, or an acceleration in a second direction relative to the vehicle.

Adding or removing or modulating the sound of a (further) musical instrument or a processed version thereof may also provide the person with an indication of the condition of the vehicle in terms of acceleration. Adding or removing the sound of a musical instrument or a processed version thereof may leave other physical properties of the audible sound (such as volume and/or frequency) the same, or may be used in addition to a modification in terms of such other physical properties.

As merely one example, the sound of a drum roll could be generated/added when the vehicle passes over an uneven road surface, such as an unsurfaced road (gravel road or similar), where a significant amount of vertical acceleration (up and down, in particular in close succession) would be expected to be experienced.

As one other example, in a steady state of the vehicle (travelling in a straight line at a constant speed), the sound of string instruments could be generated by the vehicle, and when an acceleration (e.g. a positive acceleration in a forward direction) is detected, the sound of another instrument could be added, e.g. a percussion instrument or a brass instrument.

In some examples, that are not part of the claimed invention, the acoustic output device comprises, or forms part of, one or more loudspeakers of the vehicle, in particular a surround sound system of the vehicle, in particular with a noise cancellation function.

Such loudspeakers would typically be permanently installed in the vehicle. Pursuant to the present example, surround sound systems installed in the vehicle may comprise as few as three loudspeakers, but preferably more, for example at least 4, 5, 6, 7, 8, 9 or 10 loudspeakers. They may have (significantly) more than ten loudspeakers, for example at least 15, 20, 25 or 30.

In the embodiment of the invention, the acoustic output device comprises, or forms part of, a portable device, in particular a portable device that is proprietary to the vehicle, or a personal device, in particular a smartphone, a laptop computer, a tablet computer, headphones or a headset.

Whether the acoustic output device is proprietary to the vehicle or not, it (or portions thereof) may be removable (from the vehicle). For example, in the case of a device proprietary to the vehicle, the vehicle may include a holder or cradle or similar for carrying the portable device. Such a cradle may, for example, be provided on the back of a headrest (of a front seat) for use by a person sitting on a seat behind that headrest. However, other locations are also possible. When the portable device is placed in the holder or cradle, it is substantially fixed with respect to the vehicle. However, it can be removed from the holder or cradle (and may or may not still be attached to the vehicle via a cable or similar). It may be proprietary to the vehicle in the sense that it may have proprietary connectors which (typically) only fit a corresponding connector of the holder or cradle, or the communication protocols of the holder or cradle (of the vehicle) need to match those of the portable device, so that a generic device (such as a personal mobile device) might not be able to communicate with the vehicle via the holder or cradle.

There are examples, that are not part of the claimed invention, in which no holder or cradle is provided in connection with a proprietary device. Again, the device may be proprietary to the vehicle in the sense that the communication protocols of the portable device need to match those of the vehicle in order to be able to communicate with the vehicle, whereas a generic device (such as a personal mobile device) might not be able to communicate with the vehicle.

Whether the acoustic output device is proprietary to the vehicle or is a (generic) personal device, it may be connected to the vehicle in a wired or wireless manner, for example via Bluetooth^{®} or similar.

In the embodiment of the invention in which the acoustic output device comprises a portable device, the processing unit is arranged to cause the acoustic output device to output the audible sound in such a way that a user of the acoustic output device perceives the audible sound as coming from a substantially consistent direction and/or location relative to the vehicle, irrespective of the location and/or orientation of the acoustic output device relative to the vehicle.

These embodiments are based on the following recognition by the inventor: If an audible sound is output in order to reduce the effects of motion sickness, this can be particularly effective if the audible sound is output (or perceived to come) from a consistent direction with respect to the vehicle. For example, if the audible sound is intended to imitate the sound of an internal combustion engine which, in many vehicles in use today, is located near the front of the vehicle, in a central position, the system can be set up such that the audible sound is output in such a way that the sound is perceived by a user as coming from a central position at the front of the vehicle.

This will briefly be illustrated by way of example. We assume that a user is sitting on the front right-hand seat of the vehicle, is facing forward (in the longitudinal direction of the vehicle) and is wearing a headset with surround sound function. The central position at the front of the vehicle might be at a position which corresponds to a direction, with respect to that user, at 11 o'clock (assuming that 12 o'clock corresponds to a direction straight ahead, 9 o'clock corresponds to a direction to the left and so on). Now, if, for example, the user turns their head 90° to the left, the central position at the front of the vehicle might now be at a position which corresponds to a direction, with respect to that user, at 2 o'clock. In order to ensure that the user can perceive the audible sound as coming from a consistent direction, with respect to the vehicle, the system can take the position and/or orientation of the portable device - here the headset of the user - into account. To this end, in some embodiments, the system comprises an interface for receiving information regarding the position and/or orientation of the portable device relative to the vehicle, and the processing unit is arranged to generate the control signal as a function of this information. Using this control signal, the portable device is then "instructed" accordingly, i.e. such that the user can perceive the audible sound as coming from the central position at the front of the vehicle.

In such embodiments, the portable device could itself provide the information regarding the position and/or orientation of the portable device relative to the vehicle, for example using gyroscopes of the portable device and/or positional information obtained with the aid of a (satellite-based) positioning system such as GPS. This information can then be processed by the system, in particular the processing unit. In particular, the system/processing unit can compare this information with information regarding the position and/or orientation of the vehicle so as to determine the position and/or orientation of the portable device relative to the vehicle. In addition, or as an alternative, the vehicle could obtain the information regarding the position and/or orientation of the portable device relative to the vehicle using e.g. one or more cameras or other detectors (installed in the vehicle) arranged to detect the position and/or orientation of the portable device.

The interface for receiving information regarding the position and/or orientation of the portable device relative to the vehicle may be wired or wireless. A wired interface may, for example, be an electrical connector for connection to the portable device. If the portable device is hard-wired to the processing unit, the interface may be regarded as a point along the connection between the processing unit and the portable device.

In alternative embodiments, the processing unit provides, to the portable device, information regarding the direction/position (with respect to the vehicle) which the audible sound is supposed to be perceived to originate from. This information is provided to the portable device without the processing unit necessarily being aware of the position and/or orientation of the portable device relative to the vehicle. Using this information, the portable device can then determine, on the basis of its own knowledge of its own position and/or orientation, how to generate and output the audible sound so that the audible sound is able to be perceived by the user as coming from the "correct" direction/position.

In the sense of the invention, the expressions "correct direction/position" and "substantially consistent direction relative to the vehicle" and similar are preferably intended to be understood in such a way that an angular difference between, on the one hand, the direction which the audible sound is supposed to be perceived to originate from, and, on the other hand, the direction from which the audible sound will be perceived, by a user, to be coming from, is at most, or less than, one of: 90°, 80°, 70°, 60°, 50°, 45°, 40°, 30°, 20° or 10°.

In some embodiments, the acceleration comprises any one, or all, or any subset, of longitudinal acceleration, vertical acceleration, lateral acceleration, a right curve, a left curve or road unevenness.

For this purpose, the system may be (permanently) (pre)configured in a corresponding manner, or such that it can be (re)configured, in particular by a user. For example, a user might decide that the system should only output the audible sound in case of longitudinal acceleration and curves to the right and left, but not for other types of acceleration (vertical acceleration or road unevenness) and can adjust the system accordingly. It is also conceivable that different users can adjust settings of the system according to their individual needs, in particular if at least one user uses a portable device such as a headset or headphones. That is, the first user may for example specify (and configure the system accordingly) that an audible sound is provided to the first user for all types of acceleration, whereas a second user may for example specify (and configure the system accordingly) that an audible sound is provided to the second user only for a subset of these types of acceleration.

In some embodiments, the processing unit is arranged to classify the type of acceleration, in particular using a classification algorithm. This makes it easier to distinguish between the different types of acceleration such as longitudinal acceleration, vertical acceleration, lateral acceleration, a right curve, a left curve or road unevenness.

In some embodiments, the processing unit is further configured to generate the control signal in dependence upon the type of acceleration detected by the detector, in particular on the basis of the classification by the processing unit.

For example, the processing unit might be configured such that the control signal to be generated by the processing unit, in the case of (positive) longitudinal acceleration, will cause the volume and frequency of the audible sound to be output by the acoustic output device to increase, whereas, in the case of the vehicle passing through a left or right curve, the audible sound to be output by the acoustic output device will be perceived as coming from the left or right, respectively.

As another example, the classified type of acceleration (which the vehicle - as well as the occupants - experience or are subjected to) can be used by the processing unit to cause a particular type of audible sound to be generated (or not or no longer to be generated) or the sound of a particular (musical) instrument or a processed version thereof to be generated (or not or no longer to be generated). Or, expressed in a different way, the classified type of sound (or instrument as part of the sound) may be made dependent on the classified type of acceleration.

This being by way of example only, it will be appreciated that other configurations will be possible.

In some embodiments, the detector is further configured to detect an upwards and/or a downwards movement of the vehicle and to provide a further detection output based thereon;
wherein the processing unit is configured to receive the further detection output, the processing unit further being configured to generate a further control signal based thereon; and
wherein the acoustic output device is configured to receive the further control signal and to output the audible sound as a function of the further control signal.

These embodiments are based on the following recognition by the inventor: If a vehicle is going uphill or downhill - even if a vertical component of the velocity of the vehicle is not changing (over time), or even if the vehicle is travelling (uphill or downhill) in a straight line at a constant speed (i.e. in the absence of any acceleration of the vehicle, other than gravity) - a user may sense that the vehicle (and consequently the seat on which the user is sitting) is at a non-zero angle with respect to an orientation when the vehicle is travelling in a horizontal plane. This may contribute towards the user developing motion sickness. Hearing an audible sound as an indication of the uphill or downhill movement of the vehicle may help to reduce the effects of motion sickness.

At least some of the features described above in connection with acoustic output devices with surround sound function can - albeit in a more limited sense - also be implemented in acoustic output devices with a stereo function - both where these acoustic output devices form part of the vehicle or where they comprise, or form part of, a portable device. For example, a lateral acceleration can conveniently be audibly indicated to a user by outputting an audible sound via an acoustic output device respectively on the left or right-hand side of the user (e.g. left or right-hand speaker of headphones worn by the user, or left or right-hand loudspeaker of the vehicle).

In a second aspect, the present disclosure provides a vehicle for road use comprising the system according to the first aspect, or any embodiment thereof.

In a third aspect, the present disclosure provides a method of providing an indication of an acceleration of a vehicle for road use, the method comprising:
detecting an actual acceleration of the vehicle and providing a detection output based thereon;
processing the detection output and generating a control signal based thereon; and
outputting an audible sound as a function of the control signal, the audible sound being indicative of the actual acceleration of the vehicle.

In a fourth aspect, the present disclosure provides a computer program product comprising a program code which is stored on a computer readable medium, for carrying out the method in accordance with the third aspect, or any of its steps or combination of steps, or any embodiments thereof.

The computer program may in particular be stored on a non-volatile data carrier. Preferably, this is a data carrier in the form of an optical data carrier or a flash memory module. This may be advantageous if the computer program as such is to be traded independently of a processor platform on which the one or more programs are to be executed. In a different implementation, the computer program may be provided as a file or a group of files on one or more data processing units, in particular on a server, and can be downloaded via a data connection, for example the Internet, or a dedicated data connection, such as for example a proprietary or a local network. In addition, the computer program may comprise a plurality of interacting, individual program modules.

In some embodiments, the computer program may be updatable and configurable, in particular in a wireless manner, for example by a user or manufacturer. Likewise, the audible sound (to be generated) or the manner in which it is generated or modified, in particular the conditions which need to be fulfilled so that the audible sound will be generated or modified, may be updatable and configurable in some embodiments, in particular in a wireless manner, for example by a user or manufacturer.

The terms "first", "second", "third" and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Where the term "comprising" or "including" is used in the present description and claims, this does not exclude other elements or steps. Where an indefinite or definite article is used when referring to a singular noun, e.g. "a", "an" or "the", this includes a plural of that noun unless something else is specifically stated.

Further, unless expressly stated to the contrary, "or" refers to an inclusive "or" and not to an exclusive "or". For example, a condition "A or B" is satisfied by any one of the following: A is true (or present) and B is false (or not present), A is false (or not present) and B is true (or present), and both A and B are true (or present).

All connections mentioned in the present specification may be wired or wireless unless this is explicitly excluded or technically impossible. The term "wired connection" encompasses not only connections established by, or using, a (metallic) wire, but also optical connections, preferably also any other type of physical connection allowing for the transmission of information.

Aspects and embodiments of the invention described or claimed herein can be combined with each other, and embodiments described in connection with one aspect of the invention can be combined with other aspects of the invention, unless such a combination is explicitly excluded or technically impossible.

Further advantages, features and applications of the present invention are provided in the following detailed description and the appended figures, wherein:
- **Fig. 1**: schematically shows a plan view of a vehicle according to an embodiment of the present invention.
- **Fig. 2**: shows a flow chart illustrating a method according to an embodiment of the present invention.
- **Fig. 3**: schematically shows a top view of the head of a person wearing headphones, in a first orientation.
- **Fig. 4**: schematically shows a top view of the head of the person from Fig. 3, in a second orientation.

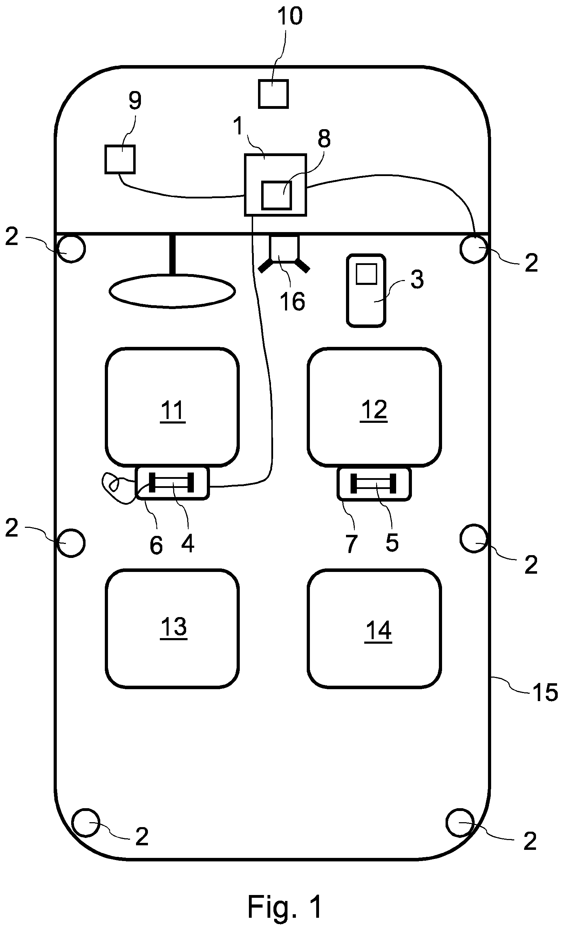

**Fig. 1** schematically shows a plan view of a vehicle 15 according to an embodiment of the present invention. The vehicle 15 is shown, by way of example, as a left-hand drive car, with two front seats 11, 12, two rear seats 13, 14 and a steering wheel (not labelled). The front of the vehicle is at the top of Fig. 1.

Vehicle 15 is equipped with a processing unit 1. Processing unit 1 may comprise, or form part of, an onboard computer of vehicle 15.

Processing unit 1 is connected to one or more loudspeakers 2 which are distributed at locations in the vehicle 15 and may be substantially permanently installed in vehicle 15. In the example shown in Fig. 1, there are six such loudspeakers 2. Only one (wired) connection between processing unit 1 and one of the loudspeakers 2 (right-hand side, towards the front of the vehicle 15) is shown. The other loudspeakers 2 may be connected in like manner. The connection(s) may be wired or wireless. More, or fewer, than six loudspeakers 2 may be provided. With only one loudspeaker 2, only a mono effect may be achievable. With two loudspeakers 2, a stereo effect may be achievable. For a surround sound effect, at least three loudspeakers 2 may be necessary, although it is preferred that vehicle 15 is equipped with (significantly) more than three loudspeakers. Loudspeakers 2 may all be positioned at the same height within the vehicle 15, or at different heights, in which case a user may perceive the output from the loudspeakers 2 as coming not only from locations within one (horizontal) plane but also from locations above and/or below.

Processing unit 1 is also connected to one or more detectors 9 of a type that is able to detect a current, actual acceleration of vehicle 15. Detector(s) 9 may, for example, comprise an accelerometer and/or a gyroscope.

Additionally, processing unit 1 is connected to one or more portable devices. Fig. 1 illustrates three such portable devices 3, 4, 5. These will now be explained.

Portable device 3 is a personal portable device, such as a smartphone, laptop or tablet computer, headphones or a headset or similar, any of which may be connected to vehicle 15, in particular to processing unit 1, in a wired or wireless manner, for example via Bluetooth^{®} or similar.

Portable device 4 is proprietary to vehicle 15. It is held by, placed upon, or (removably) attached to a cradle or holder 6, which may be (substantially permanently) installed in vehicle 15, for example attached to the back of seat 11 or the headrest of seat 11, and may form part of a rear seat entertainment (RSE) system. Cradle 6 is connected to processing unit 1 by a wired connection. Portable device 4 is also connected to cradle 6 by a wired connection. Portable device 4 may comprise a loudspeaker, headphones or a headset and may have a surround sound function. The location of cradle 6 with respect to the vehicle 15 may be known to processing unit 1. A possible use of this information will be described later.

Portable device 5 and cradle/holder 7 may substantially correspond to portable device 4 and cradle/holder 6, except that these are not connected by a wired connection to processing unit 1, i.e. there is neither a wired connection between processing unit 1 and cradle 7, nor between cradle 7 and portable device 5. Portable device 5 may communicate with cradle 7, or directly with processing unit 1, in a wireless manner.

Cradle 7 may also communicate with processing unit 1 in a wireless manner, or may not communicate with processing unit 1 at all and may simply be a holder for (mechanically) accommodating portable device 5 and/or serve as a charging station for portable device 5.

Variations of portable devices 4, 5 and cradles/holders 6, 7 are also possible, for example such that the portable device 4, 5 is connected to a respective cradle 6, 7 in a wireless manner whilst the respective cradle 6, 7 is connected to processing unit 1 by a wired connection. Alternatively, portable device 4, 5 may be connected to the respective cradle 6, 7 by a wired connection whilst the respective cradle 6, 7 is connected to processing unit 1 in a wireless manner.

Processing unit 1 may have one or more wireless interfaces 8 for communicating with any one, some or all of loudspeakers 2, personal device 3, portable devices 4, 5 and/or detector 9. Signals may be sent between processing unit 1 and portable devices 3, 4, 5 via their respective connections, if applicable via cradles 6, 7.

In addition or as an alternative, processing unit 1 may comprise a wired interface, for example an electrical connector for a cable-based connection with any, some or all of the other devices mentioned above. If these devices are hard-wired to processing unit 1, then the interface may be regarded as a point along the connection between these devices and processing unit 1. A wired connection can also be an indirect wired connection, for example such that cradles 6, 7 are connected to processing unit 1 via a first wired connection (hard-wired or using a removable cable with one or more connectors), and portable devices 4, 5 are connected to their respective cradle 6, 7 via a second wired connection (hard-wired or using a removable cable with one or more connectors, for example plugged into an AUX socket of the respective cradle).

In some embodiments, not all of the devices are present or connected, or the system of certain embodiments of the invention may comprise only some of the devices illustrated in Fig. 1. For example, embodiments are possible where the system comprises only the processing unit 1, detector 9 and one audible output device such as a loudspeaker 2 or one of the portable devices 3-5.

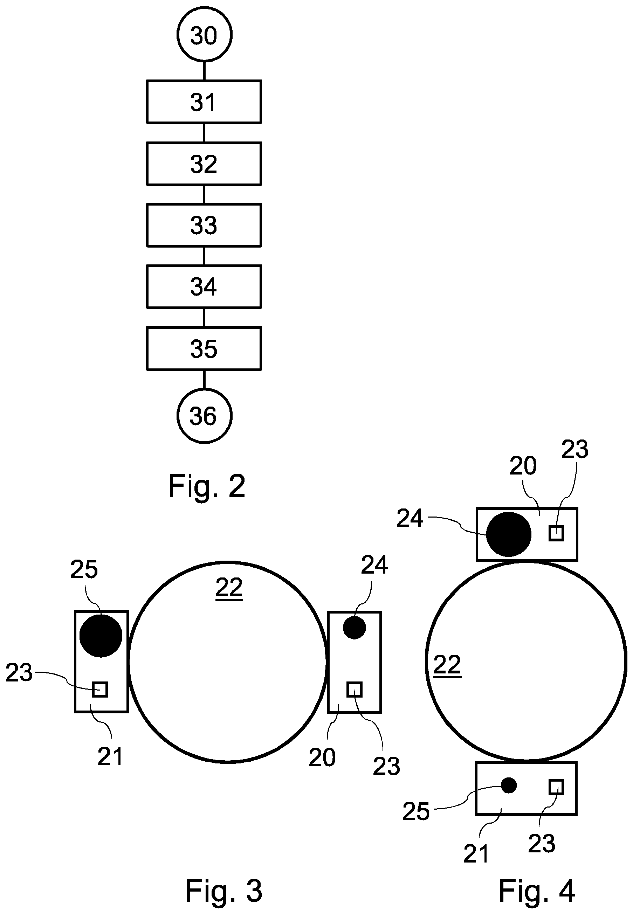

A method of operation of a system in accordance with the present invention will now be described with continued reference to Fig. 1 and further with reference to Fig. 2.

**Fig. 2** shows a flow chart illustrating a method according to an embodiment of the present invention.

After the start (30) of the method, the detector 9 detects (31) an actual (current) acceleration of the vehicle and provides (32) a detection output based thereon. The acceleration can be any one, or all, or any subset, of longitudinal acceleration, vertical acceleration, lateral acceleration, a right curve, a left curve, or road unevenness.

The detection output is then transmitted to processing unit 1, where the detection output is processed (33) or analysed. In particular, processing unit 1 may include circuitry or program components to amplify the detection output, or to distinguish between detection outputs associated with the different types of acceleration, and to generate (34) a control signal based on the detection output. The control signal is thus indicative of the acceleration of the vehicle 15.

One or more of the acoustic output devices 2-5 then receives the control signal and generates and outputs (35) an audible sound as a function of the control signal. The audible sound is thus indicative of the actual acceleration of the vehicle 15. The user is thus given an audible indication of the current, actual acceleration of vehicle 15, which may reduce the effects of motion sickness. Once the audible sound has been output, the method may end (36), or alternatively may be repeated.

As an (optional) additional step (not illustrated in Fig. 2), the location of one or more of the output devices 3-5 with respect to vehicle 15 may be determined. In the case of loudspeakers 2, their location would typically be fixed with respect to vehicle 15, and their location may be known to processing unit 1 in advance, e.g. stored in a memory device of processing unit 1, so that a determination of their location does not need to be carried out.

Various ways of generating the audible sound as a function of the control signal are possible. For example, the volume or frequency of the audible sound can be modulated with, or as a function of, the control signal. As one example, if the control signal is proportional to, or varies linearly with, the detected acceleration and if the audible sound is modulated as a function of the control signal in such a way that the volume of the audible sound is proportional to, or varies linearly with, the control signal, then the volume of the audible sound will also be proportional to, or will vary linearly with, the detected acceleration. As another example, the frequency of the audible sound could be modulated as a function of the control signal in such a way that the frequency of the audible sound varies linearly with the control signal and thus with the detected acceleration.

Of course, other relationships, in particular non-linear relationships, are possible between (a property of) the audible sound and the control signal/the detected acceleration. Likewise, depending on a measure of the acceleration, or the type of acceleration, different sounds may be added/removed, e.g. sounds of different (musical) instruments or processed versions thereof. Further, depending on a measure of the acceleration, or the type of acceleration, the processing unit 1 may cause the audible sound to be output from one or more particular acoustic output devices, e.g. such that the number of loudspeakers 2 from which the audible sound is to be output increases with increasing acceleration.

In some embodiments, the system can be configured such that an audible sound is generated whenever the vehicle 15 is operational, for example after a key or key card has been inserted into a corresponding receptacle or a start button has been pressed. Alternatively, the audible sound can be generated whenever the vehicle 15 is moving. In particular, the audible sound may resemble the sound of an internal combustion engine. The control signal can then be used by the system to modify a property of the audible sound.

One possibility of modifying a property of the audible sound is this: the audible sound may be composed of the sound of one or more musical instruments or processed versions thereof. When an acceleration has been detected, in particular above a fixed or (user) configurable threshold, the sound of a further musical instrument may be added to the audible sound. Similarly, the sound of a musical instrument may be omitted/removed from the audible signal, for example on detection of a deceleration.

The audible output devices 3-5 may also have a noise cancellation function. Similarly, vehicle 15 may have a noise cancellation function. In this case, outputting an audible sound indicative of the actual acceleration of vehicle 15 may be particularly useful in reducing the effects of motion sickness since vehicles or audible output devices with noise cancellation function - for example when a user is listening to music - tend to cause a user to be even less aware of surrounding noises such as an engine noise, which tends to contribute to motion sickness.

In some embodiments, the processing unit 1 can distinguish between different types of acceleration, such as longitudinal acceleration, vertical acceleration, lateral acceleration, a right curve, a left curve, or road unevenness. For example, the control signal may vary dependent on the type of acceleration detected. For example, in the case of a positive longitudinal acceleration, i.e. a positive acceleration in a forward direction, the control signal may be such that the audible sound is predominantly or exclusively output from acoustic output devices located towards the front of the vehicle 15, whereas in the case of a negative longitudinal acceleration, i.e. a deceleration, the control signal may be such that the audible sound is predominantly or exclusively output from acoustic output devices located towards the rear of the vehicle 15. Similarly, for left and right curves, the audible sound could predominantly or exclusively be output from acoustic output devices respectively located towards the left and right of vehicle 15. In a case of road unevenness, the audible sound could be output by acoustic output devices in such a way that it is perceived, by a user, to come from a location towards the bottom of the vehicle 15.

The different types of acceleration can also be distinguished in terms of the type of audible sound to be output, e.g. modulation in terms of volume/frequency for longitudinal acceleration and adding/removing a musical instrument for lateral acceleration etc.

The embodiment of the invention will now be described with continued reference to Figs. 1 and 2 and further with reference to Figs. 3 and 4.

Fig. 3 schematically shows a top view of the head 22 of a person wearing headphones (or a headset), in a first orientation, and Fig. 4 schematically shows a top view of the head 22 of the person from Fig. 3, in a second orientation. The reference number 22 is respectively placed towards the direction in which head 22 is facing. In other words, in the orientation of Fig. 3, the head 22 is turned towards the top of the figure, whereas in Fig. 4, the head 22 is turned towards the left.

Figs. 3 and 4 schematically show headphones worn by the person, whereby reference number 20 indicates a right-hand portion of the headphones and reference number 21 indicates a left-hand portion of the headphones. In the embodiment shown, the headphones 20, 21 are headphones with surround sound function. The headphones are further equipped with one or more (built-in) devices 23 for determining the orientation/position of the headphones, in particular with respect to a local or global reference. Such devices 23 can, for example, comprise a gyroscope and/or an accelerometer, in particular a 3-axis accelerometer, for example one gyroscope/accelerometer per headset or, as shown in Fig. 3, one gyroscope/accelerometer 23 each for the right-hand portion 20 and the left-hand portion 21 of the headphones. Such devices 23 can, for example, (also) determine the orientation/position of the headphones with respect to a satellite-based signal, such as a GPS signal.

We now assume that the person (whose head 22 is shown in Figs. 3 and 4) is sitting on the front right-hand seat 12 of vehicle 15. The person shown in Fig. 3 is initially facing in the direction of travel of vehicle 15, i.e. towards the front of vehicle 15. We further assume that the system described above with reference to figure 1 is intended to generate and output an audible sound indicative of the actual acceleration of the vehicle 15. Further, in this example, we assume that the person is supposed to perceive the audible sound as coming approximately from a central location at the front of vehicle 15, where, at least in most conventional vehicles with an internal combustion engine, the engine would typically be located. This location is indicated as a square 10 in Fig. 1. In accordance with this embodiment, the audible sound will be likely to provide the person with a particularly realistic impression of a sound coming from an internal combustion engine - even if vehicle 15 is an electric vehicle, for example.

In relation to the head 22 of the person, the location 10 may be considered to correspond to a direction of 11 o'clock (12 o'clock being straight ahead). In order for the system to generate the audible sound in such a way that the person will perceive it as coming from the direction 11 o'clock, the system determines, using gyroscopes and/or accelerometers 23 or similar, the orientation and/or position of the headphones 20, 21 in relation to vehicle 15. On determining that the person is facing straight ahead, the system causes the headphones 20, 21 to output the audible sound in such a way that the person will perceive the audible sound as coming (approximately) from the central location 10 at the front of vehicle 15, i.e. the direction 11 o'clock with respect to the head 22 of the person. In Fig. 3, this is indicated by a larger black circle 25 (indicating a higher volume) towards the front of the left-hand portion 21 of the headphones and a smaller black circle 24 (indicating a lower volume) towards the front of the right-hand portion 20 of the headphones.

Referring now to Fig. 4, the person has turned their head 22 by 90° towards the left. The central location 10 at the front of vehicle 15 now corresponds to 2 o'clock with respect to the head 22 of the person. Again using information (provided by gyroscopes and/or accelerometers 23 or similar) regarding the (changed) orientation/position of the headphones, the system can cause the headphones 20, 21 to output the audible sound in such a way that the person will perceive the audible sound as coming (approximately) from the central location 10 at the front of vehicle 15, i.e. the direction 2 o'clock with respect to the head 22 of the person. In Fig. 4, this is indicated by a larger black circle 24 (indicating a higher volume) slightly towards the front of the right-hand portion 20 of the headphones and a very small black circle 25 (indicating a very low volume - lower than the volume 24 in Fig. 3) slightly towards the front of the left-hand portion 21 of the headphones.

It will be appreciated that the technique illustrated with reference to Figs. 3 and 4 can be used with other locations/directions. However, it is preferred that the audible sound is perceived by the person as coming from a consistent direction with respect to vehicle 15, regardless of the orientation/position of headphones 20, 21.

It is preferred that the audible sound is perceived by the person not only as coming from a consistent direction with respect to vehicle 15 but also as coming from an appropriate/consistent distance. We consider again the example of the audible sound imitating an engine noise coming from a central location 10 at the front of vehicle 15. Further, in this example, we assume that a first user is sitting on (front) seat 12, and a second user is sitting on (rear) seat 14, behind seat 12. An embodiment of the invention envisages that the first and second users not only perceive the audible sound as coming from slightly different directions, but also that the second user perceives the audible sound as coming from a location at a greater distance than is the case for the first user. Accordingly, assuming the (approximate) location of the acoustic output devices respectively used by the first and second users has been determined or is already known (to processing unit 1), processing unit 1 can take this information into account to generate appropriate control signals respectively for the acoustic output devices used by the first and second users to ensure that the first user will perceive the audible sound as coming from a closer distance than the second user. It is envisaged that in most cases this will mean (inter alia) that the audible sound which the first user will perceive is at a greater volume than the audible sound that the second user will perceive.

Ensuring that the "correct" portion of headphones 20, 21 is activated and at an appropriate volume so as to enable the person to perceive the audible sound as coming from a consistent direction can be implemented in at least two ways, which will be explained in the following.

In the first of these implementations, the gyroscopes and/or accelerometers 23 or similar provide information regarding the orientation/position of the headphones 20, 21 to the processing unit 1. The processing unit 1 then generates the control signal taking into account this information, i.e. the control signal then includes information as to which portion of headphones 20, 21 to activate and at what volume (based on the distance between headphones 20, 21 and the consistent location 10). The headphones 20, 21 can then use the received control signal to output the audible sound 24, 25. The headphones 20, 21 then do not need to calculate themselves which portion of headphones 20, 21 to activate and at what volume (taking into account the direction from which the audible sound is supposed to be perceived as coming from, as well as the orientation/position of the headphones 20, 21, and in particular the distance between headphones 20, 21 and the consistent location 10) - since the control signal received from processing unit 1 already contains this information.

In the second of these implementations, the gyroscopes and/or accelerometers 23 or similar again provide information regarding the orientation/position of the headphones 20, 21. However, this information is not transmitted to the processing unit 1 so that the processing unit then generates the control signal without being aware of the orientation/position of headphones 20, 21. The control signal will therefore also not include information as to which portion of headphones 20, 21 to activate and at what volume but instead informs the headphones 20, 21 of the position (with respect to vehicle 15) from which the audible sound is supposed to be perceived as coming from. The headphones 20, 21 can then use the received control signal and the information provided by its own gyroscope(s) and/or accelerometer(s) 23 or similar to calculate which portion of headphones 20, 21 to activate and at what volume so that the person will perceive the audible sound as coming from the "correct"/consistent direction with respect to vehicle 15 and "correct"/consistent distance.

Techniques for determining the location of an acoustic output device such as headphones 20, 21 with respect to a reference, such as vehicle 15 or a particular reference location within the vehicle 15, are known per se. For example, if an acoustic output device such as headphones 20, 21 are connected to processing unit 1 or a cradle 6, 7 or some other reference point of vehicle 15 via Bluetooth^{®} or similar, signal parameters such as the angle of arrival, potentially detected at multiple, spaced apart locations, can be used to determine the relative location of the acoustic output device.

In a variant, the orientation/position of the headphones 20, 21 is detected by one or more cameras or other sensors, such as camera 16 shown in Fig. 1, and the corresponding information is then provided to processing unit 1 and/or the headphones 20, 21. Camera 16 may be substantially permanently installed in vehicle 15. The position of camera 16 in Fig. 1 is indicative only; other locations are possible.

In a further development of any of the embodiments described above, the system also detects whether the vehicle 15 is travelling uphill or downhill, for example using detector (gyroscope/accelerometer) 9, and/or using a global reference such as a signal from a satellite-based positioning system. Even in the absence of any acceleration (other than gravity), a (further) control signal indicative of the uphill or downhill movement can then be generated and a corresponding audible sound can be output based thereon, in accordance with what has been described above in connection with detected acceleration of vehicle 15.

While at least one example embodiment of the present invention has been described above, it has to be noted that a great number of variations thereto exist. Furthermore, it is appreciated that any described example embodiments only illustrate non-limiting examples of how the present invention can be implemented and that it is not intended to limit the scope, the application or the configuration of the apparatuses and methods described herein. Rather, the preceding description will provide the person skilled in the art with instructions for implementing at least one example embodiment of the invention, whereby it has to be understood that various changes of functionality and the arrangement of the elements of the example embodiment can be made without deviating from the subject-matter defined by the appended claims.

### List of Reference Signs

- 1: processing unit
- 2: acoustic output devices / loudspeakers / surround sound system
- 3: portable / personal device
- 4: wired acoustic output device / headphones
- 5: wireless acoustic output device / headphones
- 6: wired cradle / holder
- 7: wireless cradle / holder
- 8: (wireless) interface
- 9: detector
- 10: central position at front of vehicle
- 11 - 14: seat
- 15: vehicle
- 16: camera / detector
- 20: headphones (right-hand portion)
- 21: headphones (left-hand portion)
- 22: head of user
- 23: position and/or orientation determination devices / gyroscopes / accelerometer
- 24: right side output
- 25: left side output
- 30 - 36: method steps

## Claims

1. A system (1, 2-5, 9) for providing an indication of an acceleration of a vehicle (15) for road use, the system (1, 2-5, 9) comprising:
a detector (9) configured to detect an actual acceleration of the vehicle (15) and to provide a detection output based thereon;
a processing unit (1) configured to receive the detection output, the processing unit (1) further being configured to generate a control signal based thereon; and
an acoustic output device (2-5) configured to receive the control signal and to generate and output an audible sound (24, 25) as a function of the control signal, the audible sound (24, 25) being indicative of the actual acceleration of the vehicle (15),
wherein the acoustic output device (2-5) comprises, or forms part of, a portable device (3-5), and wherein the processing unit (1) is arranged to cause the acoustic output device (2-5) to output the audible sound (24, 25) in such a way that a user of the acoustic output device (2-5) perceives the audible sound (24, 25) as coming from a substantially consistent direction (10) and/or location (10) relative to the vehicle (15), irrespective of the location and/or orientation of the acoustic output device (2-5) relative to the vehicle (15).

2. The system (1, 2-5, 9) according to claim 1, wherein the system (1, 2-5, 9) is configured to modulate the audible sound (24, 25) with, or as a function of, the control signal.

3. The system (1, 2-5, 9) according to claim 1 or 2, wherein the system (1, 2-5, 9) is configured to modulate the audible sound (24, 25) with, or as a function of, the control signal in terms of frequency and/or volume.

4. The system (1, 2-5, 9) according to any one of the preceding claims, wherein the audible sound (24, 25) is an audible sound (24, 25) arranged to be generated by the vehicle (15)
- to indicate that the vehicle (15) is operational and/or
- to indicate that the vehicle (15) is moving and/or
- to resemble the sound of an internal combustion engine.

5. The system (1, 2-5, 9) according to any one of the preceding claims, wherein the audible sound (24, 25) is composed of a sound of one or more musical instruments or processed versions thereof, wherein the processing unit (1) is preferably configured to modify the sound to be output, by the acoustic output device (2-5), by:
- adding a sound of a further musical instrument or a processed version thereof upon detection of an acceleration of the vehicle (15), in particular an acceleration surpassing a predetermined or configurable threshold, in particular an acceleration of the vehicle (15) in a first direction relative to the vehicle (15), and/or
- removing the sound of a musical instrument or a processed version thereof upon detection of a deceleration of the vehicle (15), in particular a deceleration surpassing a predetermined or configurable threshold, or an acceleration in a second direction relative to the vehicle (15).

6. The system (1, 2-5, 9) according to any one of the preceding claims, wherein the portable device (3-5), is a portable device (4, 5) that is proprietary to the vehicle (15), or a personal device (3), in particular a smartphone (3), a laptop computer, a tablet computer, headphones (20, 21) or a headset (20, 21).

7. The system (1, 2-5, 9) according to any one of the preceding claims, wherein the acceleration comprises any one, or all, or any subset, of longitudinal acceleration, vertical acceleration, lateral acceleration, a right curve, a left curve, or road unevenness.

8. The system (1, 2-5, 9) according to claim 7, wherein the processing unit (1) is arranged to classify the type of acceleration, in particular using a classification algorithm.

9. The system (1, 2-5, 9) according to any one of the preceding claims, wherein the processing unit (1) is further configured to generate the control signal in dependence upon the type of acceleration detected by the detector (9), in particular on the basis of the or a classification by the processing unit (1).

10. The system (1, 2-5, 9) according to any one of the preceding claims, wherein the detector (9) is further configured to detect an upwards and/or a downwards movement of the vehicle (15) and to provide a further detection output based thereon;
wherein the processing unit (1) is configured to receive the further detection output, the processing unit (1) further being configured to generate a further control signal based thereon; and
wherein the acoustic output device (2-5) is configured to receive the further control signal and to output the audible sound (24, 25) as a function of the further control signal.

11. A vehicle (15) for road use comprising the system (1, 2-5, 9) according to any one of the preceding claims.

12. A method of providing an indication of an acceleration of a vehicle (15) for road use, the method comprising:
detecting (31) an actual acceleration of the vehicle (15) and providing (32) a detection output based thereon;
processing (33) the detection output and generating (34) a control signal based thereon; and
outputting (35) an audible sound (24, 25) as a function of the control signal, the audible sound (24, 25) being indicative of the actual acceleration of the vehicle (15),
wherein outputting (35) the audible sound (24, 25) comprises outputting (35) the audible sound (24, 25):
- via an acoustic output device (2-5) which comprises, or forms part of, a portable device (3-5), and
- in such a way that a user of the acoustic output device (2-5) perceives the audible sound (24, 25) as coming from a substantially consistent direction (10) and/or location (10) relative to the vehicle (15), irrespective of the location and/or orientation of the acoustic output device (2-5) relative to the vehicle (15).

13. A computer program product comprising a program code which is stored on a computer readable medium, for carrying out a method in accordance with claim 12, when run on a system according to any of claims 1 to 10.

## Patentansprüche

1. System (1, 2-5, 9) zum Bereitstellen einer Angabe einer Beschleunigung eines Fahrzeugs (15) für den Straßenverkehr, wobei das System (1, 2-5, 9) umfasst:
einen Detektor (9), der dazu ausgelegt ist, eine tatsächliche Beschleunigung des Fahrzeugs (15) zu detektieren und eine Detektionsausgabe basierend darauf bereitzustellen;
eine Verarbeitungseinheit (1), die dazu ausgelegt ist, die Detektionsausgabe zu empfangen, wobei die Verarbeitungseinheit (1) ferner dazu ausgelegt ist, ein Steuersignal basierend darauf zu erzeugen; und
eine akustische Ausgabevorrichtung (2-5), die dazu ausgelegt ist, das Steuersignal zu empfangen und einen hörbaren Ton (24, 25) als eine Funktion des Steuersignals zu erzeugen und auszugeben, wobei der hörbare Ton (24, 25) die tatsächliche Beschleunigung des Fahrzeugs (15) angibt,
wobei die akustische Ausgabevorrichtung (2-5) eine portable Vorrichtung (3-5) umfasst oder ein Teil dieser ist, und wobei die Verarbeitungseinheit (1) so eingerichtet ist, dass sie bewirkt, dass die akustische Ausgabevorrichtung (2-5) den hörbaren Ton (24, 25) auf eine solche Weise ausgibt, dass ein Benutzer der akustischen Ausgabevorrichtung (2-5) den hörbaren Ton (24, 25) als von einer im Wesentlichen konsistenten Richtung (10) und/oder einem im Wesentlichen konsistenten Ort (10) relativ zu dem Fahrzeug (15) kommend wahrnimmt, ungeachtet des Ortes und/oder der Orientierung der akustischen Ausgabevorrichtung (2-5) relativ zu dem Fahrzeug (15).

2. System (1, 2-5, 9) nach Anspruch 1, wobei das System (1, 2-5, 9) dazu ausgelegt ist, den hörbaren Ton (24, 25) mit dem Steuersignal, oder als eine Funktion desselben, zu modulieren.

3. System (1, 2-5, 9) nach Anspruch 1 oder 2, wobei das System (1, 2-5, 9) dazu ausgelegt ist, den hörbaren Ton (24, 25) mit dem Steuersignal, oder als eine Funktion desselben, hinsichtlich Frequenz und/oder Lautstärke zu modulieren.

4. System (1, 2-5, 9) nach einem der vorstehenden Ansprüche, wobei der hörbare Ton (24, 25) ein hörbarer Ton (24, 25) ist, der dazu eingerichtet ist, durch das Fahrzeug (15) erzeugt zu werden,
- um anzugeben, dass das Fahrzeug (15) betriebsbereit ist, und/oder
- um anzugeben, dass sich das Fahrzeug (15) bewegt, und/oder
- um dem Geräusch eines Verbrennungsmotors zu ähneln.

5. System (1, 2-5, 9) nach einem der vorstehenden Ansprüche, wobei der hörbare Ton (24, 25) aus einem Ton eines oder mehrerer Musikinstrumente oder verarbeiteter Versionen davon besteht, wobei die Verarbeitungseinheit (1) vorzugsweise dazu ausgelegt ist, den durch die akustische Ausgabevorrichtung (2-5) auszugebenden Ton durch Folgendes zu modifizieren:
- Hinzufügen eines Tons eines weiteren Musikinstruments oder einer verarbeiteten Version davon nach Detektion einer Beschleunigung des Fahrzeugs (15), insbesondere einer Beschleunigung, die eine vorbestimmte oder konfigurierbare Schwelle überschreitet, insbesondere einer Beschleunigung des Fahrzeugs (15) in eine erste Richtung relativ zu dem Fahrzeug (15), und/oder
- Entfernen des Tons eines Musikinstruments oder einer verarbeiteten Version davon nach Detektion einer Abbremsung des Fahrzeugs (15), insbesondere einer Abbremsung, die eine vorbestimmte oder konfigurierbare Schwelle überschreitet, oder einer Beschleunigung in eine zweite Richtung relativ zu dem Fahrzeug (15).

6. System (1, 2-5, 9) nach einem der vorstehenden Ansprüche, wobei die portable Vorrichtung (3-5) eine portable Vorrichtung (4, 5), die dem Fahrzeug (15) eigen ist, oder eine persönliche Vorrichtung (3) ist, insbesondere ein Smartphone (3), ein Laptop-Computer, ein Tablet-Computer, Kopfhörer (20, 21) oder ein Headset (20, 21).

7. System (1, 2-5, 9) nach einem der vorstehenden Ansprüche, wobei die Beschleunigung ein beliebiges, oder alle, oder einen beliebigen Teilsatz, von Längsbeschleunigung, Vertikalbeschleunigung, Querbeschleunigung, einer Rechtskurve, einer Linkskurve oder Straßenunebenheit umfasst.

8. System (1, 2-5, 9) nach Anspruch 7, wobei die Verarbeitungseinheit (1) dazu eingerichtet ist, die Art von Beschleunigung zu klassifizieren, insbesondere unter Verwendung eines Klassifikationsalgorithmus.

9. System (1, 2-5, 9) nach einem der vorstehenden Ansprüche, wobei die Verarbeitungseinheit (1) ferner dazu ausgelegt ist, das Steuersignal in Abhängigkeit von der Art von Beschleunigung zu erzeugen, die durch den Detektor (9) detektiert wird, insbesondere auf Basis der oder einer Klassifikation durch die Verarbeitungseinheit (1) .

10. System (1, 2-5, 9) nach einem der vorstehenden Ansprüche, wobei der Detektor (9) ferner dazu ausgelegt ist, eine Aufwärts- und/oder eine Abwärtsbewegung des Fahrzeugs (15) zu detektieren und eine weitere Detektionsausgabe basierend darauf bereitzustellen; wobei die Verarbeitungseinheit (1) dazu ausgelegt ist, die weitere Detektionsausgabe zu empfangen, wobei die Verarbeitungseinheit (1) ferner dazu ausgelegt ist, ein weiteres Steuersignal basierend darauf zu erzeugen; und wobei die akustische Ausgabevorrichtung (2-5) dazu ausgelegt ist, das weitere Steuersignal zu empfangen und den hörbaren Ton (24, 25) als eine Funktion des weiteren Steuersignals auszugeben.

11. Fahrzeug (15) für den Straßenverkehr, das das System (1, 2-5, 9) nach einem der vorstehenden Ansprüche umfasst.

12. Verfahren zum Bereitstellen einer Angabe einer Beschleunigung eines Fahrzeugs (15) für den Straßenverkehr, wobei das Verfahren umfasst:
Detektieren (31) einer tatsächlichen Beschleunigung des Fahrzeugs (15) und Bereitstellen (32) einer Detektionsausgabe basierend darauf;
Verarbeiten (33) der Detektionsausgabe und Erzeugen (34) eines Steuersignals basierend darauf; und
Ausgeben (35) eines hörbaren Tons (24, 25) als eine Funktion des Steuersignals, wobei der hörbare Ton (24, 25) die tatsächliche Beschleunigung des Fahrzeugs (15) angibt,
wobei das Ausgeben (35) des hörbaren Tons (24, 25) das Ausgeben (35) des hörbaren Tons (24, 25) umfasst:
- über eine akustische Ausgabevorrichtung (2-5), die eine portable Vorrichtung (3-5) umfasst oder Teil davon bildet, und
- auf eine solche Weise, dass ein Benutzer der akustischen Ausgabevorrichtung (2-5) den hörbaren Ton (24, 25) als von einer im Wesentlichen konsistenten Richtung (10) und/oder einem im Wesentlichen konsistenten Ort (10) relativ zu dem Fahrzeug (15) kommend wahrnimmt, ungeachtet des Ortes und/oder der Orientierung der akustischen Ausgabevorrichtung (2-5) relativ zu dem Fahrzeug (15).

13. Computerprogrammprodukt, das einen Programmcode umfasst, der auf einem computerlesbaren Medium gespeichert ist, zum Ausführen eines Verfahrens nach Anspruch 12, wenn es auf einem System nach einem der Ansprüche 1 bis 10 ausgeführt wird.

## Revendications

1. Système (1, 2-5, 9) pour produire une indication d'une accélération d'un véhicule (15) à usage routier, le système (1, 2-5, 9) comprenant :
un détecteur (9) configuré pour détecter une accélération réelle du véhicule (15) et pour produire un signal de sortie de détection en fonction de celle-ci ;
une unité de traitement (1), configurée pour recevoir le signal de sortie de détection, l'unité de traitement (1) étant en outre configurée pour générer un signal de commande en fonction de celui-ci ; et
un dispositif de sortie acoustique (2-5) configuré pour recevoir le signal de commande et pour générer et émettre un son audible (24, 25) en fonction du signal de commande, le son audible (24, 25) étant indicatif de l'accélération réelle du véhicule (15),
le dispositif de sortie acoustique (2-5) comprenant un dispositif portable (3-5), ou faisant partie de celui-ci, et l'unité de traitement (1) étant conçue pour amener le dispositif de sortie acoustique (2-5) à émette le son audible (24, 25) de telle sorte qu'un utilisateur du dispositif de sortie acoustique (2-5) perçoive le son audible (24, 25) comme provenant d'une direction (10) et/ou d'un emplacement (10) sensiblement cohérent par rapport au véhicule (15), indépendamment de l'emplacement et/ou de l'orientation du dispositif de sortie acoustique (2-5) par rapport au véhicule (15).

2. Système (1, 2-5, 9) selon la revendication 1, dans lequel le système (1, 2-5, 9) est configuré pour moduler le son audible (24, 25) avec le signal de commande, ou en fonction de celui-ci.

3. Système (1, 2-5, 9) selon la revendication 1 ou 2, dans lequel le système (1, 2-5, 9) est configuré pour moduler le son audible (24, 25) avec le signal de commande, ou en fonction de celui-ci, en termes de fréquence et/ou de volume.

4. Système (1, 2-5, 9) selon l'une quelconque des revendications précédentes, dans lequel le son audible (24, 25) est un son audible (24, 25) conçu pour être généré par le véhicule (15)
- pour indiquer que le véhicule (15) est opérationnel et/ou
- pour indiquer que le véhicule (15) est en mouvement et/ou
- pour ressembler au son d'un moteur à combustion interne.

5. Système (1, 2-5, 9) selon l'une quelconque des revendications précédentes, dans lequel le son audible (24, 25) est composé d'un son d'un ou plusieurs instruments de musique ou de versions traitées de ceux-ci, l'unité de traitement (1) étant de préférence configurée pour modifier le son devant être émis par le dispositif de sortie acoustique (2-5), par :
- ajout d'un son d'un autre instrument de musique ou d'une version traitée de celui-ci lors de la détection d'une accélération du véhicule (15), en particulier une accélération dépassant un seuil prédéterminé ou configurable, en particulier une accélération du véhicule (15) dans une première direction par rapport au véhicule (15), et/ou
- retirer le son d'un instrument de musique ou d'une version traitée de celui-ci lors de la détection d'une décélération du véhicule (15), en particulier une décélération dépassant un seuil prédéterminé ou configurable, ou une accélération dans une deuxième direction par rapport au véhicule (15).

6. Système (1, 2-5, 9) selon l'une quelconque des revendications précédentes, dans lequel le dispositif portable (3-5) est un dispositif portable (4, 5) qui est propre au véhicule (15), ou un dispositif personnel (3), en particulier un smartphone (3), un ordinateur portable, une tablette informatique, des écouteurs (20, 21) ou un casque (20, 21).

7. Système (1, 2-5, 9) selon l'une quelconque des revendications précédentes, dans lequel l'accélération comprend l'une, la totalité ou un sous-ensemble de l'accélération longitudinale, l'accélération verticale, l'accélération latérale, une courbe vers la droite, une courbe vers la gauche ou une irrégularité de la route.

8. Système (1, 2-5, 9) selon la revendication 7, dans lequel l'unité de traitement (1) est conçue pour classifier le type d'accélération, en particulier en utilisant un algorithme de classification.

9. Système (1, 2-5, 9) selon l'une quelconque des revendications précédentes, dans lequel l'unité de traitement (1) est en outre configurée pour générer le signal de commande en fonction du type d'accélération détecté par le détecteur (9), en particulier en fonction de la ou d'une classification par l'unité de traitement (1).

10. Système (1, 2-5, 9) selon l'une quelconque des revendications précédentes, dans lequel le détecteur (9) est en outre configuré pour détecter un mouvement vers le haut et/ou vers le bas du véhicule (15) et pour produire un signal de sortie de détection supplémentaire en fonction de celui-ci ;
l'unité de traitement (1) étant configurée pour recevoir le signal de sortie de détection supplémentaire, l'unité de traitement (1) étant en outre configurée pour générer un signal de commande supplémentaire en fonction de celui-ci ; et
le dispositif de sortie acoustique (2-5) étant configuré pour recevoir le signal de commande supplémentaire et pour émettre le son audible (24, 25) en fonction du signal de commande supplémentaire.

11. Véhicule (15) à usage routier comprenant le système (1, 2-5, 9) selon l'une quelconque des revendications précédentes.

12. Procédé pour produire une indication d'une accélération d'un véhicule (15) à usage routier, le procédé comprenant :
la détection (31) d'une accélération réelle du véhicule (15) et la production (32) d'un signal de sortie de détection en fonction de celle-ci ;
le traitement (33) du signal de sortie de détection et la génération (34) d'un signal de commande en fonction de celui-ci ; et
l'émission (35) d'un son audible (24, 25) en fonction du signal de commande, le son audible (24, 25) étant indicatif de l'accélération réelle du véhicule (15),
l'émission (35) du son audible (24, 25) comprenant l'émission (35) du son audible (24, 25) :
- via un dispositif de sortie acoustique (2-5) qui comprend un dispositif portable (3-5), ou forme celui-ci, et
- de telle sorte qu'un utilisateur du dispositif de sortie acoustique (2-5) perçoive le son audible (24, 25) comme provenant d'une direction (10) et/ou d'un emplacement (10) sensiblement cohérent par rapport au véhicule (15), indépendamment de l'emplacement et/ou de l'orientation du dispositif de sortie acoustique (2-5) par rapport au véhicule (15).

13. Produit de programme informatique comprenant un code de programme qui est stocké sur un support lisible par ordinateur, pour mettre en œuvre un procédé selon la revendication 12, lorsqu'il est exécuté sur un système selon l'une quelconque des revendications 1 à 10.
